# EUROPEAN PATENT APPLICATION

(11) **EP 4 520 363 A1**
(43) Date of publication of application: **12.03.2025**
(21) Application number: 23196057.6
(22) Date of filing: 07.09.2023
(51) Int. Cl.: A61M 5/142, A61M 5/168, A61M 5/172, A61M 5/145

(54) **IMPLANTABLE DRUG PUMP WITH A RESERVOIR AND A FILL LEVEL SENSING SYSTEM**

(71) Applicant: B. Braun Miethke GmbH & Co. Kg, 14469 Potsdam (DE)
(72) Inventor: Metz, Ian, 10245 Berlin (DE); Sörensen, Michael, 14482 Potsdam (DE)
(74) Representative: Tergau & Walkenhorst Intellectual Property GmbH

(57) **Abstract**

An implantable drug pump (1) with a drug container (2) and a fill level sensing system shall be cost-efficient, and shall provide reliable and accurate results under various operating conditions. According to the invention, this is achieved in that the drug container (2) comprises a cavity (8) which is enclosed by a housing (4) and a cover (6) and in which an expandable and contractible bellows (10) encloses a drug reservoir (20), the bellows (10) comprising a convoluted or folded side wall (12) fixed at the cover (6) and a bellows bottom (14) in a variable distance to the cover (6), wherein
• a permanent magnet (28) is attached directly or indirectly to the bellows (10) such that its distance to the cover (6) varies upon expansion or contraction of the bellows (10),
• a magnetic field sensor (30, 30', 30") is fixedly attached to the housing (4) or the cover (6) such that it is able to sense the strength and/or the orientation of a magnetic field caused by the magnet (28), and
• the fill level sensing system comprises an evaluation unit (32) which from an output signal of the magnetic field sensor (30, 30', 30") determines the strength and/or the orientation of the magnetic field and relates it to a volume or fill level of the drug reservoir (20).

## Description

The field of the invention is implantable drug pumps that have at least one reservoir to be filled with a drug. The drug is usually delivered via a catheter to a certain spot in the patient's body to provide a therapy. A fill level sensor is used detect the amount of a fluidic volume in the reservoir.

US 2018/0318503 A1 discloses an implantable drug pump wherein an expandible and contractible bellows encloses a drug reservoir. Different sensor types to detect the position of the bellows bottom are described: strain gauge sensor, light based sensor, sonically based sensor. The measured position is then related to a volume or fill level of the drug reservoir.

US 2012/259283 A1 illustrate a reservoir fill level detector of a medical drug pump. The reservoir fill level detector includes a coil located in a bulkhead of the pump above a propellant chamber. A recess in the propellant chamber concentric with the coil receives a magnetic block that moves in and out of the recess. The magnetic block is mounted on a lever arm. The lever arm is hingedly attached to an upper portion of the propellant chamber. Movement of a bottom portion of the reservoir moves the lever arm and changes the position of the magnetic block in the recess and relative to the coil. A processor or other circuit connected to the coil measures the current through the coil. The position of the magnetic block will cause a predictable change in the current passed through the coil. The processor may record or interpret the information in combination with memory to give a reservoir fill level based upon the measured current. Therefore, the magnetic block acts as a coil core which, depending on its position, increases or decreases due to its magnetic permeability the inductance of the coil. This change in inductance is measured and correlated with the fill level.

The objective of the invention is to provide an alternative to above-mentioned fill level sensing system in an implantable drug pump which is cost-efficient and easy to implement, and which provides reliable and accurate results under various operating conditions. Furthermore, a corresponding method of determining a volume or filling level in such a system shall be given.

Regarding the apparatus, the objective is met by an implantable drug pump according to claim 1.

Hence, the invention provides an implantable drug pump with a drug container and a fill level sensing system, the drug container comprising a cavity which is enclosed by a housing and a cover and in which an expandable and contractible bellows encloses a drug reservoir, the bellows comprising a convoluted or folded side wall fixed at the cover and a bellows bottom in a variable distance to the cover, wherein
- a permanent magnet is attached directly or indirectly to the bellows such that its distance to the cover varies upon expansion or contraction of the bellows,
- a magnetic field sensor is fixedly attached to the housing or the cover such that it is able to sense the strength and/or the orientation of a magnetic field caused by the magnet, and
- the fill level sensing system comprises an evaluation unit which from an output signal of the magnetic field sensor determines the strength and/or the orientation of the magnetic field and relates it to a volume or fill level of the drug reservoir.

In contrast to the teaching of US 2012/259283 A1, which is based on the effect that a core with a magnetic permeability exerts on the electric inductivity of a coil, the present invention proposes utilizing the long-distance effect of a magnetic field generated by magnet, such that the strength and/or the orientation of said magnetic field is measured by a suitable sensor and then related (or correlated) to a volume or fill level (e.g. half full, 10% full etc.) of the drug reservoir. This can, for example, be achieved by calibration measurements. Unlike in said prior art wherein the magnetic probe (which has a magnetic permeability but not necessarily generates a magnetic field of its own) immerses into the associated coil, the magnet may remain in some distance from the magnetic field sensor and may even be separated from it by a material barrier, provided it does not shield the magnetic field too much. This opens up new constructive possibilities for implementation. Furthermore, surprisingly accurate measurement results can be obtained with minimal energy consumption.

In a preferred embodiment, the magnetic field sensor is a Hall sensor, an AMR sensor, or a GMR sensor, or any other type of sensor that is able to detect the field strength and/or the angle of a magnetic field relative to a reference direction.

Preferably, the magnet has the following properties:

| Property | Parameter | Tolerance | SI unit |
|---|---|---|---|
| Diameter | 1.50 | ±0.05 | mm |
| Length | 1.50 | ±0.05 | mm |
| Magnetic Strength | 120 | minimum | mT |
| Base Areas | parallel | - | - |
| Edges | burr-free | - | - |

In a preferred setting the magnet is located inside the drug reservoir. In particular, the magnet may be attached to the bellows bottom such that it directly follows the displacement of the bellows bottom. Alternatively, the magnet may be attached to a sheet or bar protruding from a convolution of the side wall of the bellows such that there is a lever or gear effect, in which the displacement of the magnet is smaller than that of the bellows bottom. A similar effect may be achieved when the magnet is attached to a lever arm which is pivot-mounted between the bellows bottom and the cover. Also, the sheet or the lever arm may help to bring the magnet closer to the associated magnetic field sensor. When then magnet is located inside the drug reservoir it is preferably enclosed with a biocompatible sleeve, e.g. a titanium sleeve. Furthermore, the cover advantageously comprises a recess for accommodating the magnet during a contracted state of the bellows, such that a full contraction with (near) zero volume of the drug reservoir can be realized.

In a preferred setting, the magnetic field sensor is located outside the cavity, in particular outside the drug container. In particular, the magnetic field sensor may be attached to the cover. Alternatively, the magnetic field sensor may be attached to a bottom wall of the housing beneath the bellows. In a variation thereof, the magnetic field sensor may be located inside a sensor chamber within the housing.

In general, it is preferred that either the housing or the cover forms a material barrier between the magnet and the magnetic field sensor. This way, the delicate magnetic field sensor may be fluidically separated and sealed from the location of the magnet and from the drug to be delivered. In this case the magnetic field sensor is preferably located in a region of the housing or the cover with reduced wall thickness (in comparison with the rest of the housing or cover) in order to avoid a strong weakening of the magnetic field.

To perceive the magnetic field of the magnet in the best possible way and in great strength, the magnetic field sensor is preferably aligned colinearly with the magnet on a symmetry axis of the magnetic field.

In an advancement of the general idea there are two magnetic field sensors configured to cover different ranges of motion of the magnet and to provide respective output signals to the evaluation unit. In this case, advantageously one of the magnetic field sensors is attached to the cover, and the other one is attached to the housing.

With respect to method, the present invention provides a method of determining a volume or filling level of a drug reservoir within a drug container of an implantable drug pump, the drug container comprising a cavity which is enclosed by a housing and a cover and in which an expandable and contractible bellows encloses a drug reservoir, the bellows comprising a convoluted or folded side wall fixed at the cover and a bellows bottom in a variable distance to the cover, wherein
- a permanent magnet is attached directly or indirectly to the bellows such that its distance to the cover varies upon expansion or contraction of the bellows, and
- a magnetic field sensor is fixedly attached to the housing or the cover such that it is able to sense the strength and/or the orientation of a magnetic field caused by the magnet, and
wherein the strength and/or the orientation of the magnetic field is determined from an output signal of the magnetic field sensor and is related to a volume or fill level of the drug reservoir.

Objectives, features, and advantages related to the apparatus closely correspond to those of the method and vice versa and hence need not be repeated here.

Various embodiments of the invention are subsequently discussed with reference to the accompanying drawings.

FIG. 1 shows a sectional view of a drug container of an implantable drug pump with a bellows in an expanded state and with components of an associated filling level sensor, in particular a magnet and a magnetic field senor.

FIG. 2 shows the drug container of FIG. 1 wherein the bellows is in a contracted state.

FIG. 3 to 7 show further embodiments of an implantable drug container with associated filing level sensor.

Similar elements are designated the same reference numerals throughout the description.

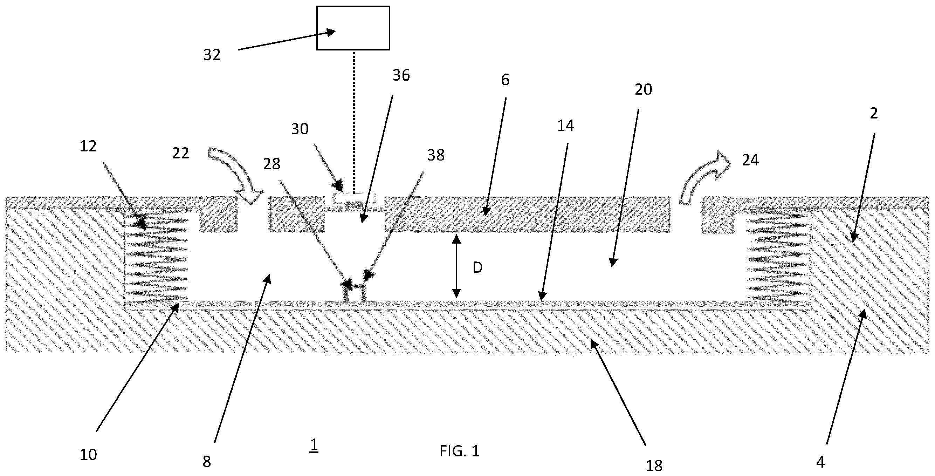

FIG. 1 shows a sectional view of a drug container 2 of an implantable drug pump 1. The drug pump 1 with the drug container 2 is implantable into a human or mammal body to deliver a controlled flow of a liquid drug contained in a reservoir of the drug container 2 to a body region of interest, usually via a catheter attached or integral to the drug pump 1. The system may also be called an intrathecal drug delivery system. Due to the implanted use, a high-level of biocompatibility for all the components in contact with the body and/or the drug is generally of paramount importance.

The drug container 2 comprises a housing 4 with a tub-like depression which is covered by a cover 6, in particular a cover plate, such that the housing 4 and the cover 6 enclose a cavity 8. In the example, the cavity 8 has a cubic shape. A bellows 10 with an expandable and contractible side wall 12 (typically forming a cylindrical enclosure) comprising a plurality of zigzag-wise folded leaves and a generally flat bellows bottom 14 is arranged within the cavity 8 such that the cavity 8 is partitioned into two fluidically separated (sealed to each other) sub-volumes. To this end, the side wall 12 is attached to and sealed against the underside of the cover 6 while the bellows bottom 14 is aligned essentially in parallel to the cover 6 in some distance D to it. Depending on the degree of expansion or contraction of the bellows 10, said distance D is variable between a minimum distance and a maximum distance. With a recessed mounting of the bellows' side wall 12 at/in the cover 6, as shown in the example, the minimum distance can be as low a zero. The maximum distance is reached either in the maximum expanded state of the bellows 10 or when the bellows bottom 14 touches the ground of the tub-like depression in the housing 4, the upper side of the housing's bottom wall 18 thereby forming a stop.

The sub-volume between the bellows 10 and the cover 6 constitutes a drug reservoir 20 with a variable volume, depending on the degree of expansion or contraction of the bellows 10. Depending on circumstances, the minimum volume can be as low as zero while the maximum volume is ultimately limited by the volume of the cavity 8. As mentioned, the drug reservoir 20 is intended to hold and provide a liquid drug or infusion solution which is typically pressurized and fills the entire sub volume which constitutes the drug reservoir 20. An inlet port 22, in particular an aperture or hole that penetrates the cover 6, facilitates filling the drug reservoir 20 with the drug, and similarly an outlet port 24, in particular an aperture or hole that penetrates the cover 6, facilitates extraction of the drug from the drug reservoir 20.

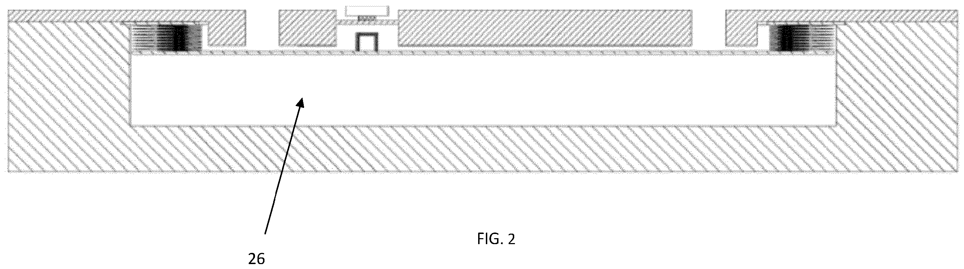

The other sub-volume between the bellows 10 and the housing 4 may constitute a propellant chamber 26 (see FIG. 2) into which during assembly (or manufacturing a pressurized fluidic propellant is inserted. When the pressure of the propellant is higher than the pressure of the drug in the drug reservoir 20, the bellows 10 is compressed or contracted and therefore the drug is pushed out of the outlet port 24.

FIG. 1 shows the maximum expanded state of the bellows 10 with maximum volume of the drug reservoir 20, whereas FIG. 2 shows the maximum contracted (or minimum expanded) state of the bellows 10 with a minimum (here: zero) volume of the drug reservoir 20 - and a maximum volume of the propellant chamber 26.

In an alternative embodiment, the cover 6 may be integral with the housing 4 or may be part of the housing 4. This may also apply to the further embodiments shown in the other figures.

To achieve a high degree of biocompatibility, the bellows 10 is preferably made of Titanium or a Titanium base alloy. Furthermore, the housing 4 and/or the cover 6 may, for example, be made of or may be based on Titanium. Most preferred are Grade 23 and Grade 5, but other Grades of Titanium may be suitable as well, for example Grades 1-4. These materials have a good biocompatibility, excellent fracture toughness and crack propagation behavior.

In any case, the displacement or distance D of the bellows bottom 14 relative to the cover 6 is (in this case: linearly) correlated to the volume of the drug reservoir 20 and therefore to the filling level of the implantable drug container 2. Hence, in a first embodiment of the invention the position or displacement or distance of the bellows 10 with respect to the fixed cover 6 or housing 4 will be used to detect the volume of the drug reservoir 20, respectively the amount of drug/fluid inside the drug reservoir 20 (generally assuming complete filling).

For this, a small permanent magnet 28 is mounted / fixed on the bellows bottom 14 and will move with it. The emitted magnetic field of the magnet 28 will be detected by a magnetic field sensor 30 on a fixed or static position. A change in volume inside the bellows 10 will change the distance between the magnet 28 and the magnetic field sensor 30. As a result, the strength and/or the angle of the magnetic field that reaches the magnetic field sensor 30 also changes with the position of the bellows bottom 10 and can be electronically related to a defined volume, e.g. by prior calibration. This evaluation is achieved in an electronic evaluation unit 32, either integrated into the magnetic field sensor 30 or arranged as an external component which is connected wire-bound or wireless to the magnetic field sensor 30. In FIG. 1 the evaluation unit 32 is indicated purely schematically, whereas in the other figures it has been omitted for simplicity.

In the example of FIG. 1 the magnet 28 is fixed to the top side of the bellows bottom 14 which faces the underside of the cover 6, protruding a little upwards, towards the cover 6. To facilitate maximum contraction of the bellows 10 towards zero volume of the drug reservoir 20, the underside of the cover 6 advantageously comprises a recess 36 to accommodate the magnet 28 during the contracted state of the bellows 10. The recess 36 may be realized by thinning the wall thickness of the cover 6 in this area.

In this embodiment the magnet 28 is positioned into the volume of the fluid, i.e. within the drug reservoir 20, and may be required to be incapsulated in a sleeve 38, preferably made of titanium or a titanium base alloy, to meet the requirement of biocompatibility. The enclosing sleeve 38 with the magnet 28 inside may be welded on the bellows bottom 14.

The magnetic field sensor 30 of this embodiment is fixed to the top side of the cover 6, i.e. outside the cavity 8 / outside the drug container 2, in particular outside the drug reservoir 20. Preferably, the magnetic field sensor 30 is mounted coaxial to the magnetic field caused by the magnet 28. In particular, the magnetic field of the magnet 28 may have a symmetry axis perpendicular to the plane of the bellows bottom 14, and the magnetic field sensor 30 may be located on said symmetry axis, i.e. above the magnet 28 in perpendicular alignment with respect to the bellows bottom plane (at "zenith" direction when viewed from the magnet 28).

In this embodiment, the cover 6 forms a material barrier between magnet 28 and magnetic field sensor 30. Preferably, the magnetic field sensor 30 sits in a region with reduced thickness of the cover 6, such that the material barrier attenuates the magnetic field of the magnet 28 which reaches the magnetic field sensor 30 as little as possible, while on the other hand maintaining mechanical integrity of the cover 6. This region of reduced wall thickness may be at the other (outer) side of a (inner) recess 36 for accommodation of the magnet 28 during contraction of the bellows 10 as discussed above.

The magnetic field sensor 30 can, for example, be a Hall sensor, AMR sensor based on the Anisotropic Magneto-Resistance (AMR) effect, GMR sensor based on the Giant Magneto-Resistance GMR) effect, or any other type of sensor that is able to detect the field strength and/or the angle of a magnetic field relative to a reference direction.

One advantage of the invention is the small and compact design of the sensing system that can be integrated in an existing product without extending its size. Furthermore, the associated method of measurement is independent on environmental conditions like changing of the temperature or change in fluid pressure. This makes it a very reliable measuring system. Depending on the chosen magnetic field sensor 30, the energy consumption can be very low. These advantages also apply to the other embodiments discussed below.

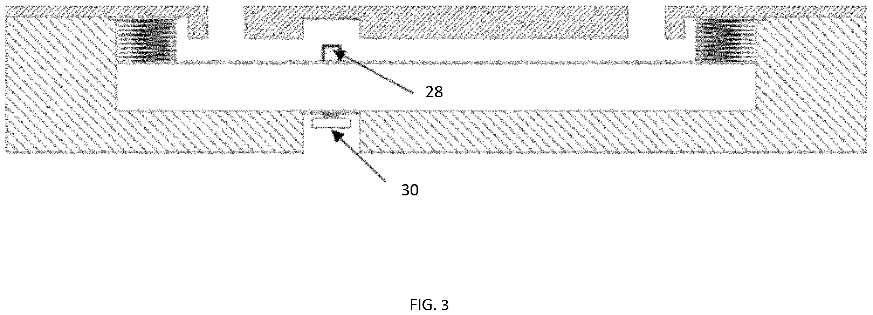

In the embodiment of FIG. 3 the magnetic field sensor 30 is mounted at the underside of a housing section below the bellows 10, outside the cavity 8. In this embodiment, the bottom wall 18 of the housing 4 beneath the magnet 28 forms a material barrier between magnet 28 and magnetic field sensor 30. Preferably, the magnetic field sensor 30 sits in a region with reduced thickness of the bottom wall 18, such that the material barrier attenuates the magnetic field of the magnet 28 which reaches the magnetic field sensor 30 as little as possible, while on the other hand maintaining mechanical integrity of the housing 4. Like in the embodiment of FIG. 1, the magnetic field sensor 30 is preferably located on a vertical through the plane of the bellows bottom 14 intersecting the magnet 28.

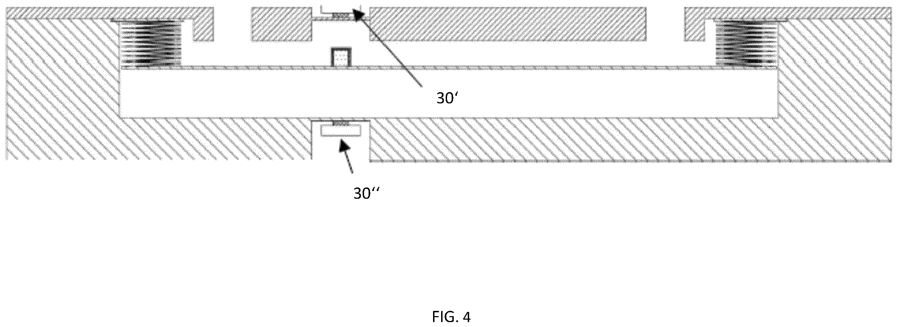

The embodiment of FIG. 4 is kind of a combination of the embodiments of FIG. 1 and FIG. 3, such that there a two magnetic field sensors 30' and 30". The first magnetic field sensor 30' is located on top side of the cover 6 as described in detail with reference to FIG. 1, and the second magnetic field sensor 30" is located beneath the bottom wall 18 of the housing 4 as described with reference to FIG. 3. Effectively, the second magnetic field sensor 30" is in coaxial alignment to the magnet 28 and the first magnetic field sensor 30'.

The benefit of this extended filling level sensing system is the higher resolution and/or the availability of the sensor data, in particular when the drug reservoir 20 approaches either the maximum expanded state (maximum displacement of the magnet 28 from the cover 6) or the maximum contracted state (maximum displacement of the magnet 28 from the bottom wall 18 of the housing 4). Thus, in the event that the magnet 28 will or might go out of range of the first magnetic field sensor 30', it has already entered the range of the second magnetic field sensor 30", and vice versa. Therefore, the whole range is covered by the totality of the two magnetic field sensors 30' and 30". Accordingly, this sensor configuration is suited for usage of a bellows 10 with a bigger volume in which a larger displacement of the bellows bottom 14 needs to be covered. In the range or portion of possible displacements which is covered by both of the sensors 30' and 30", the two sensor outputs can be compared to each other for cross-check and verification / validation. This is done by a common evaluation unit 32 (see FIG. 1) to which the two sensors 30' and 30" are connected.

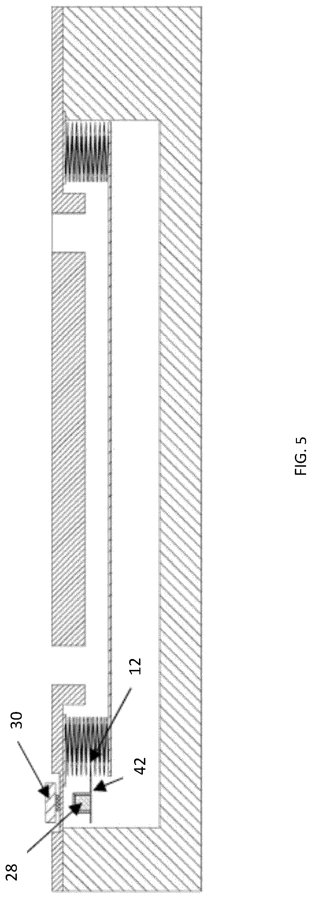

In the embodiment of FIG. 5 the magnet 28 is attached at a mid-located convolution or fold of the bellows' side wall 12 with a titanium sheet 42. The titanium sheet 42 is welded or otherwise fixed on the titanium convolution at the outer radius of the bellows 10 and supports the magnet 28. Instead of titanium another compatible material may be used for the sheet 42. With the magnet 28 therefore effectively being attached on and coupled to the displacement of the approximately mid-located convolution, the displacement of the magnet 28 is reduced (in comparison to the displacement of the bellows bottom 14) by the ratio of the number of convolutions above and below the mid-located convolution. For example, at the appropriate position the maximum displacement of the sheet-mounted magnet 28 may only be half the maximum displacement of the bellows bottom 14. This way, the magnet 28 doesn't move out range of the magnetic field sensor 30 which is aligned to the movement of the magnet 28. This embodiment allows for the usage of deeper bellows 14 with larger volume but reduces the resolution of the displacement detection and therefore volume detection of this sensor system.

In the embodiment of FIG. 5 the magnet 28 is located outside the drug reservoir 20 but within the cavity 8, namely in the second sub-volume which may constitute the propellant chamber 26. The magnetic field sensor 30 is located on the top side of the cover 6 which acts as a material barrier between magnet 28 and magnetic field sensor 30. In a variation thereof the magnetic field sensor 30 might be mounted to the underside of the housing wall 4. In a further variation the locations of magnet 28 and magnetic field sensor 30 may be reversed. All these combinations so far may be suitably combined with each other. In yet a further variation the sheet 42 supporting the magnet 28 might be located within the drug reservoir 20, pointing inwardly. That is, the magnet 28 is inside the drug reservoir 20, and the magnetic field sensor 30 is mounted on the cover 6 or below the bottom wall 18 of the housing 4.

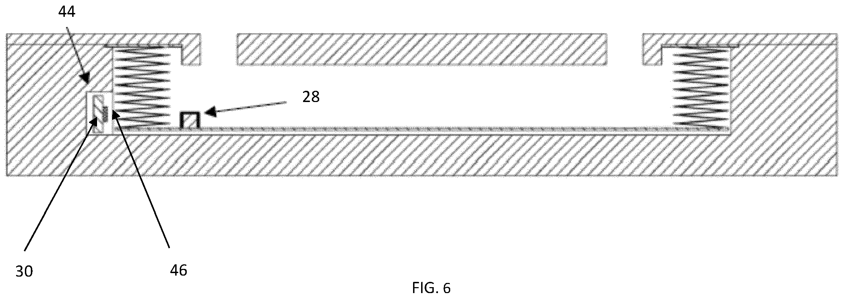

In the embodiment of FIG. 6 the magnet 28 is positioned near the edge (i.e. the side wall 12) of the bellows 10, on the bellows bottom 14, i.e. within the drug reservoir 20. The magnetic field sensor 30 is positioned in a sensor chamber 44 within the housing wall next to bellows' side wall 12 and aligned perpendicular to the direction of movement of the bellows 10. The sensor chamber 44 may be sealed against the propellant chamber 26 by a thin partition wall 46. In this embodiment the magnetic field sensor 30 detects the angle and magnitude of the magnetic field caused by the magnet 28 instead of solely the magnitude.

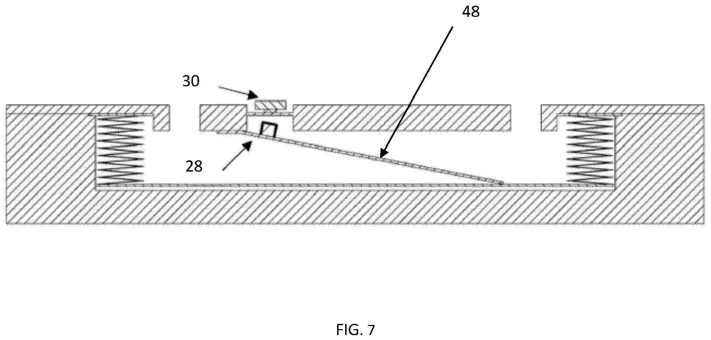

In the embodiment of FIG. 7 the magnet 28 is attached to a lever arm 48 which has contact points at the top of the drug reservoir 20 and the bottom of the bellows 10. That is, one end of the, for example, bar-shaped lever arm 48 is pivot-mounted on the bellows bottom 14, and the other end is pivot mounted on underside of the cover 6, such that in the maximum expanded state of the bellows 10 the lever arm 48 is inclined or oblique relative to the bellows bottom 14 - and lesser inclined towards the maximum contracted state of the bellows 10. In the example, the magnet 28 is attached near the upper end of the lever arm 48 to be relatively near to the magnetic field sensor 30 mounted above the magnet 28 on the top side of the cover 6. The cover 6 whose thickness may be reduced in the effective region of the magnet 28 forms a material barrier between magnet 28 and magnetic field sensor 30 - with the magnet 28 being located within the drug reservoir 20, i.e. inside the drug container 2, and the magnetic field sensor 30 being located outside the drug container 2. Similar to the embodiment of FIG. 5, the lever-mounted magnet 28 has a (in this case: linearly) lesser displacement than the bellows bottom 14. This increases the possible volume range of the drug reservoir 20 by limiting the maximum distance between the magnetic field sensor 30 and magnet 28.

In a variation of this embodiment, the magnet 28 may be attached near the lower end of the lever arm 48, i.e. near the bellows bottom 14, whereas the magnetic field sensor 30 is mounted at the underside of the bottom wall 18 of the housing 4.

Throughout the preceding description the term "drug" is generally meant to be understood in a broad sense and may include all kinds of liquid therapeutic agents, liquid medications, or infusion solutions.

Positional or directional indications like "top", "bottom", etc. have been used to conveniently designate or describe certain components or elements with reference to the position and orientation shown in the figures but do not exclude the possibility of a different orientation during usage.

### List of reference numerals

- 1: drug pump
- 2: drug container
- 4: housing
- 6: cover
- 8: cavity
- 10: bellows
- 12: side wall
- 14: bellows bottom
- 18: bottom wall
- 20: drug reservoir
- 22: inlet port
- 24: outlet port
- 26: propellant chamber
- 28: magnet
- 30, 30', 30: magnetic field sensor
- 32: evaluation unit
- 36: recess
- 38: sleeve
- 42: sheet
- 44: sensor chamber
- 46: partition wall
- 48: lever arm

- D: distance

## Claims

1. Implantable drug pump (1) with a drug container (2) and a fill level sensing system, the drug container (2) comprising a cavity (8) which is enclosed by a housing (4) and a cover (6) and in which an expandable and contractible bellows (10) encloses a drug reservoir (20), the bellows (10) comprising a convoluted or folded side wall (12) fixed at the cover (6) and a bellows bottom (14) in a variable distance to the cover (6), wherein
• a permanent magnet (28) is attached directly or indirectly to the bellows (10) such that its distance to the cover (6) varies upon expansion or contraction of the bellows (10),
• a magnetic field sensor (30, 30', 30") is fixedly attached to the housing (4) or the cover (6) such that it is able to sense the strength and/or the orientation of a magnetic field caused by the magnet (28), and
• the fill level sensing system comprises an evaluation unit (32) which from an output signal of the magnetic field sensor (30, 30', 30") determines the strength and/or the orientation of the magnetic field and relates it to a volume or fill level of the drug reservoir (20).

2. Implantable drug pump (1) according to claim 1, wherein the magnetic field sensor (30, 30', 30") is a Hall sensor, an AMR sensor, or a GMR sensor.

3. Implantable drug pump (1) according to any one of the previous claims, wherein the magnet (28) is located inside the drug reservoir (20).

4. Implantable drug pump (1) according to any one of the previous claims, wherein the magnet (28) is attached to the bellows bottom (14).

5. Implantable drug pump (1) according to any one of claims 1 to 3, wherein the magnet (28) is attached to a sheet (42) or bar protruding from a convolution of the side wall (12) of the bellows (10).

6. Implantable drug pump (1) according to any one of claims 1 to 3, wherein the magnet (28) is attached to a lever arm (48) which is pivot-mounted between the bellows bottom (14) and the cover (6).

7. Implantable drug pump (1) according to any one of the previous claims, wherein the cover (6) comprises a recess (36) for accommodating the magnet (28) in a contracted state of the bellows (10).

8. Implantable drug pump (1) according to any one of the previous claims, wherein the magnetic field sensor (30, 30', 30") is located outside the cavity (8).

9. Implantable drug pump (1) according to any one of the previous claims, wherein the magnetic field sensor (30, 30', 30") is attached to the cover (6).

10. Implantable drug pump (1) according to any one of claims 1 to 8, wherein the magnetic field sensor (30, 30', 30") is located inside a sensor chamber (44) within the housing (4).

11. Implantable drug pump (1) according to any one of claims 1 to 8, wherein the magnetic field sensor (30, 30', 30") is attached to a bottom wall (18) of the housing (4) beneath the bellows (10).

12. Implantable drug pump (1) according to any one of the previous claims, wherein either the housing (4) or the cover (6) forms a material barrier between the magnet (28) and the magnetic field sensor (30, 30', 30").

13. Implantable drug pump (1) according to any one of the previous claims, wherein the magnetic field sensor (30, 30', 30") is located in a region of the housing (4) or the cover (6) with reduced wall thickness.

14. Implantable drug pump (1) according to any one of the previous claims, wherein two magnetic field sensors (30', 30") are configured to cover different ranges of motion of the magnet (28) and to provide respective output signals to the evaluation unit (32).

15. Method of determining a volume or filling level of a drug reservoir (20) within a drug container (2) of an implantable drug pump (1), the drug container (2) comprising a cavity (8) which is enclosed by a housing (4) and a cover (6) and in which an expandable and contractible bellows (10) encloses a drug reservoir (20), the bellows (10) comprising a convoluted or folded side wall (12) fixed at the cover (6) and a bellows bottom (14) in a variable distance to the cover (6), wherein
• a permanent magnet (28) is attached directly or indirectly to the bellows (10) such that its distance to the cover (6) varies upon expansion or contraction of the bellows (10), and
• a magnetic field sensor (30, 30', 30") is fixedly attached to the housing (4) or the cover (6) such that it is able to sense the strength and/or the orientation of a magnetic field caused by the magnet (28), and
wherein the strength and/or the orientation of the magnetic field is determined from an output signal of the magnetic field sensor (30, 30', 30") and is related to a volume or fill level of the drug reservoir (20).
